Europäisches Patentamt

European Patent Office

Office européen des brevets

(11) Publication number: **0 128 041**
A2

# EUROPEAN PATENT APPLICATION

(21) Application number: 84303782.1

(22) Date of filing: 05.06.84

(51) Int. Cl.³: **C 07 G 7/00**
**A 61 K 37/02, C 12 N 5/00**

(30) Priority: 06.06.83 US 501329

(43) Date of publication of application:
12.12.84 Bulletin 84/50

(84) Designated Contracting States:
AT BE CH DE FR GB IT LI LU NL SE

(71) Applicant: Baylink, David Jeston
1534 Fern Avenue West
Redlands California 92373(US)

(72) Inventor: Baylink, David Jeston
1534 Fern Avenue West
Redlands California 92373(US)

(74) Representative: Harrison, David Christopher et al,
MEWBURN ELLIS & CO 2/3 Cursitor Street
London EC4A 1BQ(GB)

(54) Polypeptides exhibiting skeletal growth factor activity.

(57) Polypeptide compositions are provided which when introduced into a host enhance bone growth, as well as alkaline and acid phosphatase levels *in vivo*. The polypeptides may also be used in diagnostics for preparation of reagents or antibodies. The polypeptides range in molecular weight from about 10,000 to about 200,000dal.

EP 0 128 041 A2

POLYPEPTIDES EXHIBITING SKELETAL GROWTH FACTOR ACTIVITY

For several years, scientists have suspected that there was localized regulation of resorption of bone and growth of bone cells. See Farley et al., Program and Abstracts of 61st Annual Meeting of the Endocrine Society (1979); Howard and Baylink, Clinical Research (1980) 28:50A; and Howard et al., Calcif. Tissue Int. (Suppl.) (1980) 31:53 as illustrative. The possibility of there being a localized factor which controls bone growth and resorption offers the opportunity to isolate a material which could be effective in the therapeutic control of bone growth in a wide variety of situations. Such a growth factor might be useful in the treatment of osteoporosis, in conjunction with bone implants, for the production of reagents and antibodies in diagnostic assays, and in the investigation of bone growth, bone replacement and bone deterioration.

It is therefore of great importance to be able to provide compounds capable of being used for diagnosis and therapy of bone in the mammalian host.

Description of the Prior Art

Farley and Baylink, Biochemistry (1982) 21:3502-3507; and Farley et al., ibid. (1982) 21:3508-3513 describe skeletal growth factor from human bone, its isolation and characterization. See also, Baylink and Farley, Abstract of Paper given at the XVII European Symposium on Calcified Tissues, April 11-14, 1983 describing the isolation and partial purification of a putative coupling factor from human bone. Other abstracts which are cumulative of the above disclosures include Howard et al., Calcif. Tissue Int. (Suppl.) (1982) 34:S30; Murphy et al., ibid. (1982) 34:S26; Farley et al., ibid. (1982) 34:S38; and Wergedal and Baylink, *ibid.* (1982) 34:516. Urist *et al.*, *Clin.* *Orthopaedics and Rel. Res.* (1982) 162:219-231 and Sampath *et al.*: Role of extracellular matrix in local bone induction. In: Current Advances in Skeletogenesis. Eds. Silbermann and Slavkin, Amsterdam, Exerpta medica (1982) describe small factors which differ in properties or activities from the subject peptides.

### SUMMARY OF THE INVENTION

Novel polypeptides ranging over a wide molecular weight range are provided having physiological activity *in vitro* and *in vivo* as skeletal growth factors. The polypeptides are found to enhance bone cell growth, while also enhancing levels of bone and serum alkaline and serum acid phosphatase. The compounds can be used to promote the proliferation of bone cells, *in vitro* and *in vivo*, potentially in the treatment of osteoporosis, in treatment to increase longitudinal bone growth in children with short statute, and for the preparation of reagents for use in diagnosis of the level of skeletal growth factors in serum or other tissue.

### DESCRIPTION OF SPECIFIC EMBODIMENTS

Novel polypeptides of varying molecular weights are provided which when administered to bone cells induce a number of physiological effects. Bone cell proliferation occurs, which *in vivo* enhances bone formation and matrix apposition rate. Osteoclastic resorption is increased, as well as an increase in forming surface. Serum levels of alkaline phosphatase and tartrate insensitive acid phosphatase are enhanced, which increases are consistent with an increase in osteoblast number and bone formation. The polypeptides are active with vertebrates, such as mammals and birds, quadrapeds and bipeds, e.g. humans, rats, chickens, etc., as well as with fish. There appears, therefore, to be a similar mechanism in bone growth regulation over a wide spectrum of species.

The polypeptides include naturally occurring polypeptides derived from native bone, analogs of such polypeptides demonstrating one or more of the physiological characteristics of the native polypeptides and fragments thereof.

The subject polypeptides may be divided into three molecular weight categories: (1) about 100-200kdal (kilodaltons), usually 150-200kdal; (2) about 80-85kdal; and (3) about 10-20kdal.

The "high" molecular weight (150-200kdal) or large skeletal growth factor ("SGF") may be obtained from bone, particularly portions of hip bone, such as femoral heads or other portions of the hip. The bone parts are cleaned, pulverized in liquid nitrogen, demineralized and extracted with a chelating agent, e.g. ethylenediaminetetraacetic acid (EDTA), conveniently in combination with a biostatic or biocidal agent. Particularly, from about 5% to 25% EDTA with about 0.01 to 0.1% sodium azide may be employed, more particularly from about 10% to 20% EDTA and from about 0.03 to 0.05% sodium azide. Alternatively, the bone can be demineralized with HCl (0.1 to 1.0M) and extracted with aqueous solution. The extracts may then be concentrated and desalted, at least in part. Purification procedures then involve one or more of the following steps: Chromatography, e.g. on Sephadex or DEAE Sephacel; gel filtration, e.g. on Agarose, particularly Agarose 0.5M; and HPLC ion exchange chromatography.

The large SGF may be characterized by polyacrylamide gel electrophoresis (PAGE) under non-denaturing conditions, where it migrates as a single major band with bovine serum albumin tetramer, while providing multiple bands in the 50 to 90kdal range on SDS PAGE. Also, multiple bands are observed on isoelectric focusing in the acidic range, pH 4 to 6. At about physiologic pH (pH 7.2) the high molecular weight SGF binds to hydroxyapatite, QAE cellulose, and DE-52, but not CM-cellulose. The large SGF is stable to denaturing agents, such as dithiothreitol, urea and guanidine-HCl, while losing its activity upon hydrolysis with trypsin.

The "middle" molecular weight or medium SGF may be prepared in part as described above, except differing in that after treatment with EDTA, the dispersion is heated for a short period at an elevated temperature, generally in the range of about 75 to 85°C for about 15 to 30min, cooled, and insoluble protein separated and discarded. The solution is then subjected to acid precipitation (pH ~3), and the acid soluble protein neutralized to substantially neutral pH, concentrated and chromatographed (gel filtered) on Sephadex, e.g. Sephadex G-200.

The procedure may be found in greater detail in Farley and Baylink (1982), supra.

The middle molecular weight SGF gives a single band that migrates just behind bovine serum albumin monomer on PAGE under non-denaturing conditions, multiple bands on SDS PAGE in the 50-70kdal range and an isoelectric point of 6.5. The middle molecular weight SGF is stable to collagenase, β-mercaptoethanol and butanol, but loses it activity upon treatment with trypsin or urea.

The "low" molecular weight or small SGF is obtained in substantially the same manner as the large SGF. The amount of the small SGF may be enhanced by preincubation prior to HPLC gel filtration and is found to be unbound or weakly bound to DEAE Sephacel and binds to hydroxyapatite. This small SGF fraction is not observed in the preparation of human SGF, but is found in the preparation of bovine and chicken SGF. The compositions obtained from bovine and chicken are believed to contain a protease which results in the fragmentation of the large SGF to provide for the small SGF fractions. Treatment of human SGF with the protease found in other species or modifying the isolation procedure may provide for the small SGF observed with the other species.

The subject compositions can be used in a wide variety of ways, both for diagnosis and therapy. For diagnosis, the compounds may be used as antigens for the production of antibodies specific for the particular types of growth factor. The antibodies may then find application both in diagnosis and therapy. Alternatively, the polypeptides may be used as reagents in diagnostic assays, where the polypeptides may be labeled or unlabeled. Similarly, for use in diagnosis, the antibodies may be labeled or unlabeled. A wide variety of assays can be employed using one or both, the antigen or antibody, either labeled or unlabeled. Various labels include fluorescers, enzymes, chemiluminescers, radionuclides, substrates, cofactors, or the like. Assays in which the reagents may be employed include hemagglutination, radioimmunoassay, enzyme immunoassay, both homogeneous and heterogeneous, fluorescence immunoassays, substrate-linked immunofluorescence assays, or the like. These assays may be found in numerous U.S. patents, such as U.S. Patent Nos. 3,817,837; 4,133,639; 4,134,792; 4,160,016; and 4,172,117. The relevant disclosures of these patents are incorporated herein by reference as to the preparation and use of the various reagents.

The subject compounds may also be used for the study of bone-related cells in the determination of available receptors, responsive cells to the presence of the SGF, or the like. Toward this end, the subject compounds can be used in vitro for enhancing the proliferation of bone cells responsive to the SGF.

6

In situations where there is a deficiency of SGF in the serum or the patient is suffering from osteoporosis or other bone disease or when implanting bone, where it is desirable to enhance the bone proliferation in a particular area, the subject compounds can be locally administered or administered intraperitoneally, intravenously or subcutaneously. Administration of the SGF or antibodies to SGF may be in any convenient formulation in accordance with the purpose of such administration and the manner of administration. Where the protein administered will be generally distributed in the blood system, the dosage will generally be in the range of about 0.005 to about 0.5mg/g of host, depending upon the efficiency of the SGF, duration in the host and the potential for localization of the SGF in the host. The SGF may be administered neat or as a solution of from about 0.01 to 10 weight percent in a physiologically acceptable carrier, e.g. water, PBS-saline, or the like.

The subject compounds can also be used for determining receptor populations of bone cells and to modulate the growth rate of bone cells _in vitro_.

The following examples are offered by way of illustration and not by way of limitation.

EXPERIMENTAL

The preparation of human skeletal growth factor was performed as follows. Human femoral heads, (obtained at hip replacement surgery) were frozen in liquid nitrogen, crushed with a Wiley mill and repeatedly rinsed with 25mM phosphate buffer, pH 7.2, to remove all contaminating serum and marrow. The washed bone fragments were then suspended in a small volume of 10% EDTA, 0.04% sodium azide, at pH 7.2 (about 10mL/100g original weight of bone) and dialysed against the same solution at 4°C. In order to obtain the high molecular weight hSGF, the retentate is desalted with Sephadex G-25, chromatographed on DE-52

(DEAE Sephacel) and then subjected to Biogel A (0.5M) gel filtration after which time the material is treated to HPLC ion exchange chromatography followed by HPLC TSK 3000 gel filtration.

The resulting product which is found to have a molecular weight in a range from about 150,000 to 200,000dal migrates as a single major band on PAGE under non-denaturing conditions, migrating with bovine serum albumin tetramer, while multiple bands in the 50kdal to 90kdal range are observed on SDS PAGE and on isoelectric focusing in the acidic range (pH 4 to 6). At pH 7.2, the high molecular weight hSGF binds to hydroxyapatite, QAE cellulose and DEAE Sephacel, but not carboxymethyl cellulose. The large hSGF is stable to denaturing agents such as dithiothreitol, urea and guanidine HCl, while activity is lost upon treatment with trypsin.

The 83kdal hSGF is obtained by somewhat different conditions as described in Farley and Baylink (1982), supra.

Bovine skeletal growth factor (bSGF) can be prepared from the hip bones from freshly slaughtered cows. The bones are mechanically scraped clean of soft tissue and cut into 2cm³ sections. The bone sections are frozen in liquid nitrogen and ground in a Wiley mill to yield a bone powder that is washed with warm water to remove fat and serum protein, demineralized and extracted using 20% EDTA, 0.04% sodium azide as described above. The EDTA extracts are concentrated and partially desalted by Amicon ultrafiltration. The remaining EDTA is removed by desalting with Sephadex G-25. Three alternative methods can be used for further purification of the desalted crude extracts. (1) Gel filtration on Agarose yields an active bSGF fraction of 150 to 200kdal. (2) Direct purification of the crude extract on DEAE Sephacel yields a large bSGF fraction that is modestly bound. In addition, active bSGF fractions that are unbound or weakly bound to DEAE Sephacel are obtained in the range of 10 to 20kdal.

(3) Purification of desalted crude extract on hydroxyapatite yields a non-active fraction that elutes with 0.15M phosphate in an active fraction that is recovered by dissolving the hydroxyapatite support with EDTA. The tightly bound fraction yields approximately equal amounts of large bSGF and small bSGF.

The large partially purified bSGF tends to become small on further manipulation. Evidence suggests that an endogenous bone protease is present in the bone extracts and is activated during the purification process. This protease is evident in the bovine bone extracts and as will be indicated subsequently in chicken extracts, but is not observed in the purification of the human extracts.

Chicken skeletal growth factor (cSGF) is prepared from the tibia and femur of adult chickens. The bones are mechanically scraped clean of soft tissue, the cartilage ends are cut off, the bones are frozen in dry ice and smashed into 3mm$^3$ pieces. Serum proteins and fat are removed by extensive washing of the bone pieces in 0.03M tris(acetate), 0.15M NaCl, pH 7.4, with vigorous agitation. The bone is then subjected to demineralization and extraction with 10% EDTA, 0.04% sodium azide. The EDTA extracts are concentrated and partially desalted by Amicon ultrafiltration and the remaining EDTA removed by Sephadex G-25 chromatography. Two approaches are used to further purify the cSGF. (1) Gel filtration of the desalted crude extract on Agarose 0.05M yields predominately a large form of cSGF (100 to 200kdal), as well as small quantities of the small cSGF in the 10 to 20kdal range. (2) DE-52 chromatography of the desalted crude bone extract yields a cSGF fraction that is weakly bound to the matrix. The weakly bound fraction behaves like large cSGF when chromatographed on a TSK 3000 HPLC gel filtration column shortly after isolation. Preincubation of the weakly bound fraction before HPLC gel filtration results in a shift of large cSGF to the smaller cSGF. This change in apparent size is believed to be a result of the presence of a protease which is detected in the fraction weakly bound to DE-52. cSGF activity in the desalted crude extracts binds to hydroxyapatite, QAE cellulose and DE-52, but does not bind to collagen linked to Agarose or to carboxymethyl Sepharose.

Pig, salmon, and rat bones can also be processed as described above for the chicken bones. The desalted crude extracts from these three species causes a substantial increase in cellular proliferation when tested on bone cells from chick calvaria.

The extracts were then employed in a number of tests to demonstrate their activity as compared to other known growth factors as well as to determine their intrinsic activity.

The first study was a comparison of the SGF growth factors with a number of other growth factors. (See Table 1.)

TABLE 1:  Comparison of SGF with other known growth factors

| Property | GROWTH FACTOR | | | | |
|---|---|---|---|---|---|
| | SGF | EGF | FGF | IGF-1 | PDGF |
| M.W. | 150–200K[a] | 74K[c] | 13.5K | 60–150[c] | 35–38K |
| | 83K[b] | 6K | | 7–9K | |
| IEP | 4–5 | 5.4 | 6 <br> 8–9.5 | 8–8.6 | 9.8 |
| **Stability:** | | | | | |
| Heat | + | + | − | + | − |
| Acid | + | + | − | + | |
| Reducing Agents | + | − | + | − | − |
| Trypsin | − | | − | | − |
| Target Cells | Bone | Ectoderm <br> Endoderm | Mesoderm <br> 3T3 | Mesoderm <br> Fibroblast <br> 3T3, Bone | Smooth <br> Muscle |
| Source | Bone | Submaxillary <br> Gland | Pituitary <br> Brain | Liver, Serum <br> CSF | Serum <br> Platelet |

SGF:  Skeletal Growth Factor 1[a], 2[b]      EGF:  Epidermal Growth Factor[3]
FGF:  Fibroblast Growth Factor[4],[5]      IGF:  Insulin-like Growth Factor[6],[7]
PDGF: Platelet-derived Growth Factor[8]

IEP:  Isoelectric Point            c:  Exist as complex with binding proteins

0128041

TABLE 1 (continued): Comparison of SGF with other known growth factors

| | GROWTH FACTOR | | | |
|---|---|---|---|---|
| Property | ECGF | MGF | NGF | TGF |
| M.W. | 75K | 45-54K | 140K | 30-60K |
| | 17-25K | 18-23K | 26-44K | 10K |
| IEP | 4-6 | Acidic | | |
| Stability: | | | | |
| Heat | + | + | | + |
| Acid | - | | - | + |
| Reducing Agents | | | | - |
| Trypsin | | + | | - |
| Target Cells | Endothelial 3T3 | Lung Fibro- | Nerve | |
| Source | Hypothalamus | Lung | Salivary Submaxillary Gland | Transformed Cells |

EDGF: Endothelial Cell Growth Factor[9]   MGF: Macrophage Growth Factor[10,11]
NGF: Nerve Growth Factor[12]   TGF: Transforming Growth Factor[13,14]
IEP: Isoelectric Point

0128041

## References for Table 1

1[a]. Mohan et al., Unpublished observations.

2[b]. Farley, J.R. and Baylink, D.J.: Purification of a skeletal growth factor from human bone. Biochemistry (1982) 21:3502-3507.

3. Cohen, S. and Taylor, J.M.: Epidermal Growth Factor: Chemical and biological characterization. Recent Prog. Horm. Res. (1974) 30:533-550.

4. Gospodarowiscz: Purification of a Fibroblast Growth Factor from Bovine Pituitary. J. Biol. Chem. (1975) 250(7):2515-2520.

5. Lemmon et al., Bovine Fibroblast Growth Factor: Comparison of brain and pituitary preparations. J. Cell Biol. (1982) 95:162-169.

6. Hall, K. and Van Wyk, J.J., Somatomedin In: Current topics in experimental endocrinology. James, V.H. and Martin, L. Eds., Vol. 2, New York, Academic Press (1974).

7. Daughaday et al., Studies on rat somatomedin In: Somatomedins and growth. Giordano, G., Van Wyk, J.J. and Minuto, F. Eds., New York, Academic Press (1979).

8. Antoniades et al., Purification of human platelet-derived growth factor. Proc. Natl. Acad. Sci. (1979) 76(4):1809-1813.

9. Maciag et al., High and low molecular weight forms of endothelial cell growth factor. J. Biol. Chem. (1982) 257(10):5333-5336.

10. Burgess et al., Purification and properties of colony stimulating factor from mouse lung-conditioned medium. J. Biol. Chem. (1977) 252:1998-2003.

11. Stanley et al., Factors regulating macrophage production and growth. Identity of colony-stimulating factor and macrophage growth factor. J. Exp. Med. (1976) 143:631-647.

12. Koroly, M.J. and Young, M., Nerve growth factor In: Tissue Growth Factor. Baserga, R. Ed., Handbook of Experimental Pharmacology, Vol. 57, New York, Springer Verlag (1981).

13. Todaro et al., Sarcoma growth factor and other transforming peptides produced by human cells: Interactions with membrane receptors. Fed. Proc. (1982) 41(13):2996-3003.

14. Ozanne et al., Cells transformed by RNA and DNA tumor viruses produce transforming factors. Fed. Proc. (1982) 41(13):3004-3007.

The above Table 1 compares a number of physical properties of the various growth factors showing similarities and differences between the various growth factors.

In the next study, the SGF was tested in combination with a number of other growth factors or mitogens on 3T3 cells using $^3$H-thymidine incorporation as a measure of cell proliferation. The following Table 2 indicates the results.

Table 2: Effects of Combination of hSGF with Other Polypeptide Growth Factors

| Factor | % of hSGF Activity | | Significance |
| | −hSGF | +hSGF | |
|---|---|---|---|
| Control | 9.4 ± 2.4 | 100.0 ± 19 | p<0.001 |
| EGF (10ng/ml) | 13.7 ± 3.0 | 92.0 ± 27 | p<0.001 |
| FGF (10ng/ml) | 70.9 ± 13.2 | 136.6 ± 46.6 | p<0.01 |
| IGF (25ng/ml) | 30.2 ± 4.6 | 84.3 ± 13.7 | p<0.001 |
| PDGF(20ng/ml) | 34.8 ± 2.3 | 75.3 ± 9.9 | p<0.001 |

3T3 cells were plated at 70,000 cells/well in DMEM in 24 well culture dishes. 24 hours after plating, the factors were added and incubated for an additional 24 hours. The cells were then pulse labeled for 2 hours with 2μCi of $^3$H-TdR and TCA precipitable radioactivity was quantitated. Incorporation is expressed as % of maximum incorporation stimulated by hSGF alone and is the average of 6 replicates.

Comparisons are made between the maximum stimulation of the same cells with hSGF alone to the maximum stimulation by the four mitogens in the presence and absence of hSGF. hSGF alone stimulates cell proliferation seven-fold greater than EGF, three-fold greater than IGF-I and PDGF, and 40% greater than FGF. In addition, the maximum stimulation of the four mitogens was significantly elevated by the addition of hSGF. These results indicate that hSGF is non-competitive and different from the four mitogens tested.

In a similar experiment, stimulation of chick embryonic calvarial cell proliferation by fetal calf serum at different serum concentrations (0.05%, 0.1%, 0.5%, 1.0%, and 2.0%) was significantly elevated by the addition of hSGF. The maximum stimulatory effect of serum (1050% ± 133% of control with 1.0% added serum) increased by 27% (p<0.001) in the presence of hSGF.

The above results suggest that hSGF is distinct from the other known growth factors that were tested and from the growth promoting agents in the serum (polypeptide growth factors as well as fibronectin and transferrin).

Experiments were carried out _in_ _vivo_ with rats. Rats were given intraperitoneally daily injections of bovine bone extract containing 2.5mg of protein for a total of ten days. Biochemical and quantitative histological comparisons were made between the control rats and rats treated with bovine bone extract. The results of this experiment are summarized in Table 3.

Table 3

Experiment 1:  A comparison of several parameters in control rats
versus rats treated with bovine bone extract.

| Parameter | Control | Treated[a] | p[b] |
|---|---|---|---|
| Serum Calcium (mg%) | 9.8(±0.7) | 9.0(±0.5) | NS[c] |
| Body Weight (Start) | 104(±3.7) | 105(±4.2) | NS |
| Body Weight (End) | 147(±4.9) | 147(±9.2) | NS |
| Femur Alkaline Phosphatase (mU/mg protein) | 4.9(±0.9) | 11.6(±1.2) | <0.001 |
| Femur Acid Phosphatase Tartrate Insensitive (mU/mg protein) | 3.4(±0.4) | 4.8(±0.3) | <0.02 |

a  All data reported as mean ±S.E.M. and based on observations from 5 rats in the control group and 3 rats in the treated group.

b  Comparisons of treated versus control were by the students T test.

c  Not significant at the p<0.05 student T test.

Table 3 (continued)

Experiment 1:  Quantitative histological comparisons of femur and
vertebral sections from control rats and rats treated
with bovine bone extract.

| Parameter | Control | Treated[a] | p[b] |
|---|---|---|---|
| Tibiae Periosteal Bone Formation (mm$^3$) | 0.23(±0.02) | 0.40(±0.02) | <0.001 |
| Tibiae Periosteal Bone | 6.75(±0.43) | 8.60(±0.46) | <0.05 |
| Tibiae Medullary Cavity (mm$^3$) | 0.67(±0.03) | 0.81(±0.09) | <0.05 |
| Vertebral Forming Surface (single + double label) % total surface | 58.3 (±2.5) | 90.5 (±1.7) | <0.001 |
| Vertebral Neutral Surface (no label) % total surface | 41.7 (±1.9) | 9.5 (±1.4) | <0.001 |

a  All data reported as mean ±S.E.M. and based on observations from 5 rats
in the control group and 3 rats in the treated group.

b  Comparisons of treated versus control were by the students T test.

c  Not significant at the p<0.05 student T test.

This experiment was performed with five rats in the control group and three rats in the treated group. Serum calcium and final body weight were not altered by the treatment. The femur alkaline phosphatase level, a probable index of bone formation, and the tartrate insensitive acid phosphatase, a possible index of osteoclastic cell number, were both increased in the treated rats. Quantitative histological comparisons of the femur clarified the results of the enzymatic analysis. Tibia periosteal bone formation as well as the matrix apposition rate were clearly increased in the treated rats. Tibia medullary area, an index of osteoclastic resorption, was also increased in the treated rats. In addition, histological analysis of the vertebral sections gave a highly significant increase in forming surface of the treated rats.

The next study was a more detailed study on the effects of bovine bone extract _in vivo_. In this study, ten rats were used in both the control and treated groups. (See Table 4.)

## Table 4

Experiment 2: A comparison of several parameters in control rats versus rats treated with bovine bone extract.

| Parameter | Control[a] | Treated[a] | p[b] |
|---|---|---|---|
| Serum Calcium (mg%) | 10.7(±0.3) | 11.1(±0.3) | NS[c] |
| Serum Phosphate (mg/dl) | 4.7(±0.5) | 5.8(±0.2) | <0.05 |
| Serum Alkaline Phosphatase (mU/mg protein) | 63.7(±2.2) | 91.7(±5.2) | <0.001 |
| Body Weight (Start) | 86.4(±0.2) | 85.0(±0.6) | NS |
| Body Weight (End) | 149.8(±2.6) | 147.7(±2.5) | NS |

a All data reported as mean ±S.E.M. and based on observations from at least 10 rats in each group.

b Comparisons of treated versus control were by the students T test.

c Not significant at $p < 0.05$.

Table 4 (continued)

Experiment 2: Skeletal alkaline phosphatase and tartrate insensitive acid phosphatase levels in the femur, skull and sternum from control rats and rats treated with bovine bone extract.

| | Alkaline Phosphatase[a] (mU/gm bone wet weight) | | | Acid Phosphatase[a] Tartrate Insensitive (mU/gm bone wet weight) | | |
|---|---|---|---|---|---|---|
| | Control | Treated | p | Control | Treated | p |
| Femur | 155($\pm$24) | 284($\pm$19) | <0.001 | 31($\pm$5) | 60($\pm$7) | <0.001 |
| Skull | 405($\pm$44) | 1075($\pm$84) | <0.001 | 355($\pm$42) | 607($\pm$52) | <0.001 |
| Sternum | 46($\pm$2) | 93($\pm$7) | <0.001 | 107($\pm$16) | 137($\pm$14) | NS |

a  All data reported as mean $\pm$S.E.M. and based on observations from at least 10 rats in each group.

b  Comparisons of treated versus control were by the students T test.

c  Not significant at p<0.05.

Serum calcium and body weight did not change with treatment. However, both serum alkaline phosphatase (p<0.001) and serum phosphate (p<0.05) were significantly increased in the treated rats. The levels of alkaline phosphatase in the femur, skull and sternum of treated rats were elevated (p<0.001). Tartrate insensitive acid phosphatase was also increased in the femur and skull (p<0.001), but not the sternum. The results of the alkaline phosphatase analysis in the femur, skull and sternum are consistent with an increase in osteoblast number and bone formation. The alkaline phosphatase data agrees very well with the quantitative histology of the tibiae of treated rats, where a 60% increase (p<0.001) in the periosteal bone formation (mm³) and a 40% increase in the matrix apposition rate (μM/day) were observed.

Table 5:  Experiment 2 – Quantitative Histological Comparisons of Tibiae Sections from Control Rats and Rats Treated with Bovine Bone Extract

| Parameter | Control | Treated[a] | p[b] |
|---|---|---|---|
| Tibiae Periosteal Bone Formation (mm²) | 0.45(±0.02) | 0.72(±0.04) | <0.001 |
| Tibiae Periosteal Bone Apposition Rate (μM/day) | 8.58(±0.33) | 12.07(±0.51) | <0.001 |

a  All data reported as mean ± S.E.M. and based on observations from at least 10 rats in each group.

b  Comparisons of treated versus control were by the students T test.

The increase in tartrate sensitive acid phosphatase in the femur and skull is consistent with an increase in bone resorption.

A low molecular weight SGF (approximately 11 kdal) was isolated and purified from both human and bovine bones, as follows. Human femoral heads obtained during hip replacement surgery and bovine bone (pelvis and femur) obtained from a local abattoir were cleaned to remove the adhering soft tissue, cut into small pieces with a band saw, washed with water to remove marrow and fat, ground in a Wiley mill, washed with deionized water containing protease inhibitors to remove blood, and then treated with 4M guanidine hydrochloride (pH 7.4) with protease inhibitors to extract the soluble cellular constituents, serum proteins and soluble collagen. The resulting human bone residues were then demineralized, and the noncollagenous proteins extracted 4-5 times with 10% EDTA containing 4M guanidine hydrochloride and protease inhibitors (EDTA-guanidine).

Purification of the 11 kdal SGF from the human bone EDTA guanidine extracts was as follows. The extracts were subjected to hydroxylapatite chromatography which, in the presence of 4M guanidine, will pass the SGF while binding other noncollagenous proteins. The unbound fraction from the hydroxylapatite chromatography was then subjected to high pressure liquid chromatography (HPLC) on a preparative gel filtration column. Using 4M guanidine hydrochloride in 30mM Tris acetate (pH 7.4) as an eluent, it was found that the 11 kdal SGF eluted after myoglobin (17.5 kdal) and before aproteinin (6.2 kdal). The HPLC fraction containing the 11 kdal SGF was then desalted and purified by HPLC reverse phase chromatography using a silica-based $C_4$-bonded phase column which separates proteins based on their hydrophobicity. Proteins bound to the silica were eluted by increasing the concentration of acetonitrile in the eluent

-22-

(water:trifluoroacetic acid:acetonitrile). The above extraction and purification procedures are specifically for the human skeletal growth factor. Purification of the bovine skeletal growth factor involved only minor modifications of the procedure used for the human factor.

The purity of the 11 kdal human SGF was measured at each stage of purification, and the results are set forth in Table 6 below.

Table 6: Human Skeletal Growth Factor Purification Table[*]

| Step | Protein (Mg) | Specific Activity (Units/µg Protein) | Activity Recovered (%) |
|---|---|---|---|
| Guanidine EDTA | 322 | 14 | 100 |
| Hydroxylapatite (Fast Flow) | 168 | 25.5 | 95 |
| HPLC Gel Filtration | 43.5 | 75.0 | 72 |
| HPLC Reverse Phase I | 1.86 | 945 | 39 |
| HPLC Reverse Phase II | 0.21 | 4000 | 18.6 |

[*] 360 g of human femoral head bones were used as starting material. Activity of SGF was assayed by its effect on bone cell proliferation. SGF stimulates the incorporation of $^3$H-Thymidine (Tdr) into TCA precipitable material in embryonic chick calvaria cells. 100% increase in $^3$H-Tdr incorporation over unstimulated control was defined as one activity unit.

The homogeneity of both the human and bovine 11 kdal SGF fractions was demonstrated by the following observations. The SGF was shown to elute as a single symmetric peak upon HPLC reverse phase separation. The SGF was shown to migrate as a single band upon polyacrylamide-sodium deodecyl sulfate (SDS)-urea gel

electrophoresis which effects separation based on molecular weight. The SGF was also shown to migrate as a single band upon electrophoresis using a 5-15% polyacrylamide gradient gel where separation is effected based on differences in molecular size. Finally, the SGF was shown to migrate as a single band upon isoelectric focusing on a 7.5% polyacrylamide gel where separation depends on differences in the isoelectric points. Thus, the material in the 11 kdal SGF fraction was shown to have uniform molecular weight, molecular size, and isoelectric point.

The molecular weight of the purified SGF species was determined to be 11±1 kdal by polyacrylamide-SDS-urea gel electrophoresis and HPLC gel filtration using 4M guanidine hydrochloride (pH 7.4) as an eluent. The large molecular weight (83 kdal) species of SGF can be dissociated into the 11 kdal species and a carrier protein in the presence of 4M guanidine hydrochloride. The isoelectric point of the 11 kdal SGF was determined to be 6.8±0.2, and was shown to be sensitive to reducing agents such as dithiothreitol. The 11 kdal SGF was shown to be sensitive to trypsin, but resistant to treatment with collagenase and with acid (0.1% trifluoroacetic acid, pH 2.5), 6M urea, and 4M guanidine hydrochloride.

The 11 kdal SGF was shown to be a very potent mitogen for bone cells with a half-maximum activity reached at 2 to 3 ng/ml. The mitogenic activity was assayed by observing the incorporation of $^3$H-Tdr into TCA precipitable material (DNA) in embryonic chick calvarial (bone) cells. hSFG at 0.1 ng/ml, the activity (expressed as a percentage incorporation of $^3$H-Tdr to that of untreated control) was approximately 150%. The activity reached a maximum of approximately 400% at between 5 and 10 ng/ml. The maximum activity did not further increase as the concentration was increased to 100 ng/ml. bSFG showed similar activities. Preliminary studies of the amino acid composition of bovine SGF indicate that the 11 kdal SGF is rich in aspartic acid, glutamic acid, leucine, and proline. The amino acid analysis is set forth in Table 7 below.

Table 7:    Amino Acid Analysis of Bovine SGF (Residues/Molecule)

| | |
|---|---|
| Lysine | 6 |
| Histidine | 3 |
| Arginine | 3 |
| Aspartic Acid | 9 |
| Threonine | 3 |
| Serine | 5 |
| Glutamic Acid | 9 |
| Proline | 6 |
| Glycine | 5 |
| Alanine | 4 |
| Valine | 5 |
| Isoleucine | 4 |
| Leucine | 7 |
| Tyrosine | 2 |
| Phenylalanine | 3 |
| Total No. of Amino Acid Residues | 75 |

Comparisons of the molecular weight, chemical characteristics, and amino acid composition of the 11 kdal SGF with other known polypeptide growth factors indicate that the 11 kdal SGF is a unique mitogen different from other known growth factors.

The 11 kdal SGF has also been demonstrated to enhance bone formation in vivo in each of four separate experiments with ten rats per group in each experiment. Eleven kdal SGF which had been purified by EDTA extraction and hydroxylapatite chromatography from a bovine bone extract was administered to groups of rats by daily intraperitoneal injection for a period of 10 days. Control rats received BSA injections, and (in order to assess the rate of bone formation) all animals were injected with tetracycline on the first and tenth days. Tetracycline is a highly fluorescent substance that tightly binds to bone as it mineralizes. Thus, only skeletal surfaces that are undergoing bone formation become fluorescent. Animals were sacrificed after the tenth day, and one tibia from each rat was histologically evaluated for bone formation, including measurements of surface fluorescence. A portion of the other tibia was extracted with a butanol solution to release alkaline phosphatase from the plasma membranes of the osteoblasts. Alkaline phosphatase is regarded as a specific marker for the osteoblasts which are bone forming cells.

The amount of tetracycline labelled bone surface was increased in the tibiae of rats that received daily injections of the 11 kdal SGF compared to the BSA controls. The percentage of increase varied from a low of approximately 140% of control for one experiment to a high of approximately 240% of control for another experiment. The rate of bone formation on the external (periosteal) surface of the tibia was shown to be greater in rats injected with the 11 kdal SGF than in controls. The skeletal alkaline phosphatase activity in the bone samples taken from rats treated with 11 kdal SGF were also shown to be increased from a low approximately 140% in one experiment to a high of approximately 210% in another experiment. Taken together, these results clearly demonstrate that the 11 kdal SGF is a potent mitogen capable of systemically enhancing bone formation in mammals.

0128041

-26-

It is evident from the above results that the novel skeletal growth factors provided by the subject invention can be used for a multitude of purposes in enhancing the proliferation of bone cells, both *in vivo* and *in vitro*, diagnosing for the presence of these skeletal growth factors in serum or other sites, and in the treatment of various bone diseases, where there is a need for stimulation of bone growth.

Although the foregoing invention has been described in some detail by way of illustration and example for purposes of clarity of understanding, it will be obvious that certain changes and modifications may be practiced.

CLAIMS:

1. A vertebrate skeletal growth factor of from about 100,000 to 200,000dal or fragment thereof, capable of stimulating a proliferation of bone cells.

2. A skeletal growth factor according to Claim 1, wherein said vertebrate is mammal, and said growth factor is of from about 150,000 to 200,000dal.

3. A skeletal growth factor according to Claim 1, wherein said mammal is human and said skeletal growth factor is of from about 150,000 to 200,000dal.

4. A skeletal growth factor according to Claim 1, wherein said skeletal growth factor is a fragment of from about 10,000 to 20,000dal.

5. A skeletal growth factor according to Claim 1, wherein said vertebrate is human.

6. A skeletal growth factor according to Claim 1, wherein said vertebrate is bovine.

7. A skeletal growth factor according to Claim 1, wherein said vertebrate is chicken.

8. A method for enhancing the proliferation of bone, said method comprising:
contacting bone cells with an effective amount of a skeletal growth factor according to Claim 1.

9. A method according to Claim 8, wherein said vertebrate is human.

10. Antibodies capable of binding to a vertebrate skeletal growth factor according to Claim 1.

11. Antibodies according to Claim 10, conjugated with a label capable of providing a detectable signal.

12. A method for determining the presence of a vertebrate skeletal growth factor according to Claim 1, which comprises:

combining a sample suspected of containing said skeletal growth factor with an antibody according to Claim 10, and determining the occurrence of binding of said antibody to said skeletal growth factor.